# EUROPEAN PATENT SPECIFICATION

(11) **EP 3 419 689 B1**
(45) Date of publication and mention of the grant of the patent: **30.09.2020**
(21) Application number: 17705677.7
(22) Date of filing: 23.02.2017
(51) Int. Cl.: A61M 5/00, A61M 5/20, A61M 5/32, A61M 5/31

(54) **MEDICAL INJECTION DEVICE**
MEDIZINISCHE INJEKTIONSVORRICHTUNG
DISPOSITIF D'INJECTION MEDICALE

(30) Priority: 25.02.2016 EP 16157328; 31.10.2016 EP 16196553
(43) Date of publication of application: 02.01.2019
(73) Proprietor: Novo Nordisk A/S, 2880 Bagsværd (DK)
(72) Inventor: INGERSLEV, Mattias, 2880 Bagsværd (DK); FREDERIKSEN, Morten Revsgaard, 2880 Bagsværd (DK); PEDERSEN, Simon Munch, 2880 Bagsværd (DK)
(86) International application number: PCT/EP2017/054213
(87) International publication number: WO 2017/144601

(56) References cited:
- WO-A1-2012/143437
- WO-A1-2015/062845
- WO-A2-2013/034984
- WO-A2-2015/090320

## Description

### THE TECHNICAL FIELD OF THE INVENTION:

The invention relates to a medical injection device for injecting a liquid drug and especially to a pre-filled injection device for apportioning a plurality of individual settable doses. The invention especially relates to such pre-filled injection device wherein the needle cannula is covered and protected by a retractable shield between subsequent injections.

The invention further relates to a method of attaching a protective cap to a housing structure of such injection device.

### DESCRIPTION OF RELATED ART:

Pen shaped injection devices for self-treatment of chronical disease having an axially retractable axially movable shield which covers and protects the needle cannula between injections are e.g. known from WO 2016/041883.

WO 2016/041883 discloses a pen shaped injection device in which the distal part of the housing structure including the axially movable shield is covered by a removable protective cap. In order to perform an injection, the user needs to physically remove this protective cap which is usually done by pulling the protective cap axially off and away from the housing structure.

It is however sometimes desirable if the axially movable shield is provided with a locking mechanism such that the axially movable shield can only be moved in the proximal direction during injection when the locking mechanism has been activated and unlocked.

WO 2016/162284 discloses a pen shaped injection device in which the distal part of the housing structure is covered by a protective cap. However, in this injection device the protective cap is distally provided with engaging means on the inner surface which engages the axially movable shield such that a rotation of the protective cap can be transformed to a similar rotation of the axially movable shield.

WO 2015090320 discloses a pen shaped injection device with a lock arrangement involving a rotation of two sleeve elements in rotational engagement.

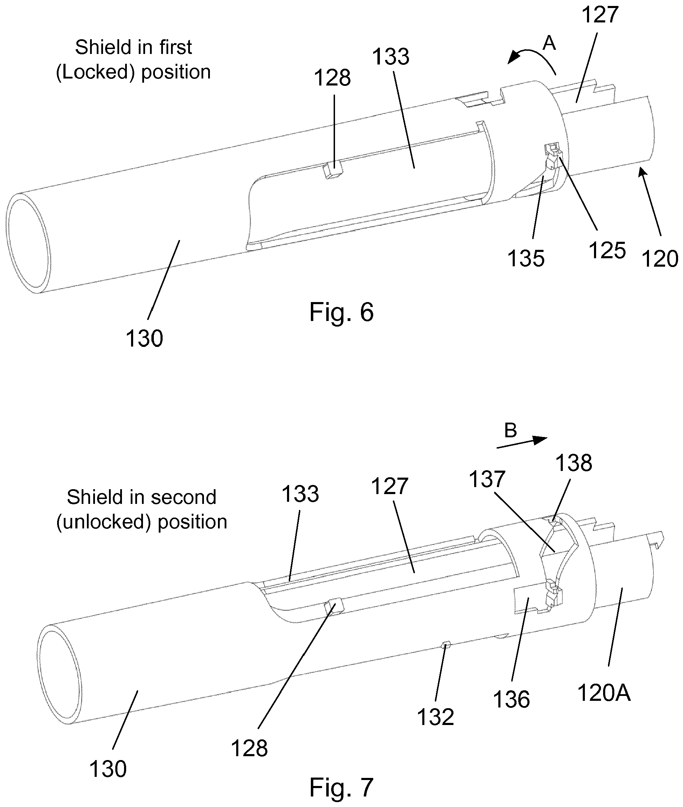

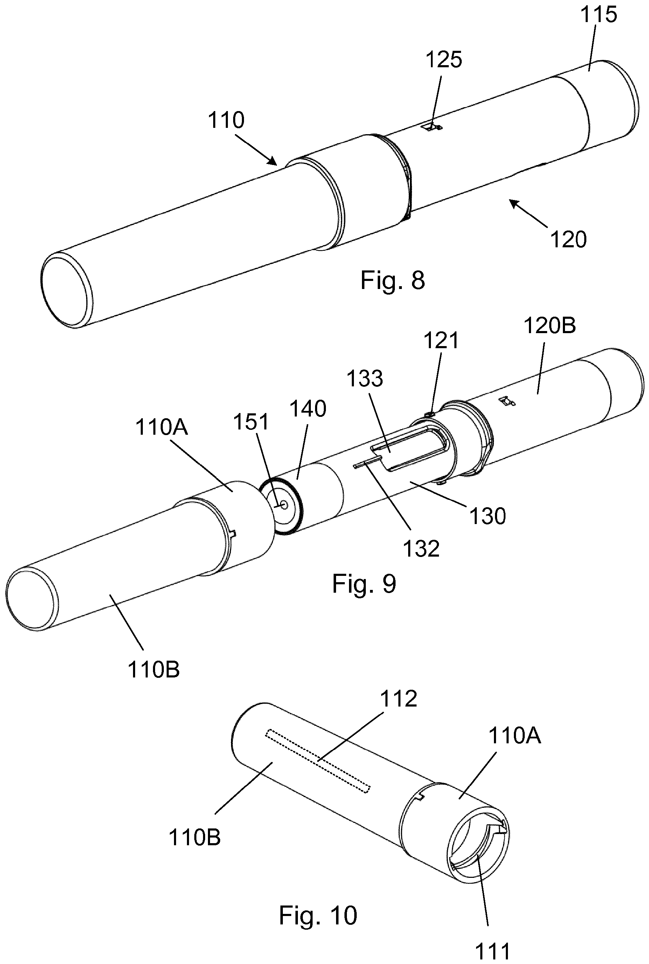

The axially movable shield is rotatable relatively to the housing structure between two different positions. In the first rotational position as depicted in figure 7, the axially movable shield is prevented from moving in the proximal direction and in the second rotational position as depicted in figure 8, the axially movable shield is allowed to move proximally.

The locked and unlocked positions are controlled by a track configuration in the axially movable shield which track configuration is rotated and moved relatively to an outwardly pointing protrusion.

The injection device is usually kept with the axially movable shield in the first and locked position between injections thereby avoiding unnecessary exposure of the needle cannula and the axially movable shield is only rotated to the second and unlocked position just before an injection is to be performed. The user thus unlocks the axially movable shield simply by rotating the axially movable shield from the first locked position to the second unlocked position.

Following an injection the user places the protective cap onto the distal part of the housing in an axial movement. However, in order to lock the axial movement of the axially movable shield the user needs to remember to rotate the axially movable shield back to the first position, either by manually rotating the axially movable shield or by rotating the protective cap engaging the axially movable shield after its mounting on the housing.

If the user forgets to rotate the axially movable shield back to the locked position there is a risk of damaging the needle cannula. This is particular cumbersome if the user either leaves the needle cannula on the injection device between injections or if the injection device is the type wherein the same needle cannula is permanently mounted and intended to be used for multiple injections.

### DESCRIPTION OF THE INVENTION:

It is an object of the present invention to provide an injection device having a shielded needle cannula which simplifies the handling of the unlocking and locking of the axial movable shield. It is in particular the intention to improve the return rotation of the axial movable shield to the locked position.

Accordingly, in one aspect, the present invention relates to a prefilled injection pen for apportioning multiple doses of a liquid drug. The injection device comprises:
- A housing structure which supports a non-removable cartridge containing the liquid drug to be ejected,
- A needle cannula having a proximal end inserted into the cartridge and a distal end with a distal tip for penetrating the skin of a user,
- An axially movable shield which covers at least the distal tip of the needle cannula between subsequent injections. This axially movable shield is rotatable relatively to the housing structure between a first position and a second positon.
   ∘ In the first position the axially movable shield is locked i.e. prevented from movements in the proximal direction and,
   ∘ In the second position, the axially movable shield is unlocked i.e. allowed to move in the proximal direction.
- A removable protective cap rotatably coupled to the housing structure and covering the distal part of the housing structure and the distal tip of the needle cannula when mounted on the housing structure.

As the protective cap is rotatably coupled to the housing structure it requires rotation of the protective cap relative to the housing in order to allow removal of the protective cap. Further, the protective cap comprises means which rotationally engages the axially movable shield such that the required rotation of the protective during removable is transformed to a rotation of the axially movable shield.

When following an injection the user mounts the protective cap onto the housing structure, the user is forced to rotate the protective cap due to its rotatable engagement with the housing structure. Via the means for engaging the axially movable shield this rotational movement of the protective cap is conveyed to a similar rotation of the axially movable shield. In this way, the axially movable shield is rotated from the second unlocked position and back to the first locked position simply by the user mounting the protective cap onto the housing structure.

Henceforth, should the user forget to lock the axially movable shield following an injection, this will be automatically done when the user mounts the protective cap on the housing structure following an injection.

The protective cap is manually removable meaning that the user of the injection device is able to both mount and dismount the protective cap by simple manipulation with the hands.

The means on the protective cap engaging the axially movable shield are provided on an inner surface of the protective cap and are formed as one or more protrusions or longitudinal structures such as ribs. Any number of such ribs can be provided but the preference is to have no less than two such ribs. The length of the ribs can also vary as long as the ribs properly engage the axially movable shield.

The axially movable shield is provided with a number of outwardly pointing protrusions or the like which is engaged by the protrusions or longitudinal ribs on the protective cap to thereby transfer rotation from the protective cap to the axially movable shield. The number of outwardly pointing protrusions preferably matches the number of longitudinal ribs. The outwardly pointing protrusions can have any suitable form.

The forced rotation of the protective cap relatively to the housing is created by a protrusion on one of the parts being guided in a track provided on the other part. In one example, the protective cap carries the inwardly pointing protrusions and the housing structure carries one or more peripheral tracks in which peripheral tracks the inwardly pointing protrusions are guided during rotation of the protective cap. However, in a different embodiment the kinematic has been reversed and the protective cap carries the peripheral track or tracks in which one or more outwardly pointing protrusions provided on the housing structure are guided.

The essential feature is that the user is forced to rotate the protective cap relatively to the housing structure in order to remove and remount the protective cap and that this rotation, at least during mounting of the protective cap, is transformed to a simultaneous rotation of the axially movable shield into its locked position thereby securing that the axial movement of the axially movable shield is not possible after mounting of the protective cap.

Further, the peripheral track connects to an axial track or opening allowing the user to fully remove the protective cap after the cap has been rotated. This final removal of the protective cap is preferably done by pulling the protective cap axially away from the housing structure.

The peripheral track can either be formed on an outer surface of the housing structure or on an inner surface of the protective cap. The peripheral track can be formed such that the protective cap is rotated purely rotationally without any axial movement during rotation or the peripheral track can be helically shaped such that the protective cap also move axially as it is rotated relatively to the housing structure.

In one example, the protective cap comprises a distal part and a proximal part which coupled together to form one operational protective cap. The parts are preferably coupled together by a simple snap- or click fit such that one part carries a counter sunk well or an opening and the other part carries a protrusion or a snap- or click arm insertable into the well or opening. However, the two parts can be connected in many alternative ways.

In this example, the peripheral track is preferably positioned in the junction between the distal part and the proximal part and preferably on an inner surface thereof.

During manufacturing, when initially mounting the protective cap onto the housing, the proximal part is first secured to the housing and the outwardly pointing protrusion is positioned in a start position in the peripheral track. Following this, the distal part is mounted by moving the distal part purely axially in relation to the housing structure. However, the distal part and the proximal part can be mounted in any random order.

When initially mounting the protective cap, the longitudinal rib can be positioned in a start position in which it will abut and rotate the axially movable shield also when the protective cap is removed for the first time. This makes it possible to use the initial rotation of the protective cap and henceforth the axially movable shield in an initiation procedure.

Such initiation procedure is in one example used to fill liquid drug from the cartridge and into a cleaning chamber which can be carried distally on the axially movable shield.

A cleaning chamber is provided so that the distal tip of the needle cannula is located inside the cleaning chamber when the axially movable shield is in the first position, and the distal tip is located outside and distal to the cleaning chamber when the axially movable shield is in the second position.

Such initiation procedure is further described in European patent application number: EP 16 188751.8 and is in one example used to pump liquid drug from the cartridge and into a cleaning chamber carried by the axially movable shield simply by an initial rotation of the protective cap.

The preservatives contained in the liquid drug are thus used as a cleaning solvent for cleaning the distal tip of the needle cannula between injections. Well known preservatives for liquid drugs are e.g. phenol or meta-cresol or a combination thereof as disclosed in WO 2015/062845.

The present invention further relates to a method of initially attaching a protective cap to a housing structure wherein the protective cap is assembled from multiple parts.

Such method comprises the steps of:
i) Initially positioning a proximal part of the protective cap relatively to the housing structure, and
ii) Moving a distal part of the protective cap axially into contact and attachment with the proximal part of the protective cap.

The proximal part is preferably mounted over the housing structure where after it is manipulated to the correct start position relatively to the outwardly pointing protrusion on the housing structure.

Once this is done the distal part is slided axially over the housing structure and into engagement with the proximal part. The connection between the two parts is preferably a snap or click connection.

### DEFINITIONS:

An **"injection pen"** is typically an injection apparatus/device having an oblong or elongated shape somewhat like a pen for writing. Although such pens usually have a tubular cross-section, they could easily have a different cross-section such as triangular, rectangular or square or any variation around these geometries.
The term **"Needle Cannula"** is used to describe the actual conduit performing the penetration of the skin during injection. A needle cannula is usually made from a metallic material such as e.g. stainless steel and preferably connected to a needle hub to form a complete injection needle, all though the needle cannula could also be connected directly to the housing structure without a needle hub. A needle cannula could however also be made from a polymeric material or a glass material.
As used herein, the term **"drug"** is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.
**"Cartridge"** is the term used to describe the container actually containing the drug. Cartridges are usually made from glass but could also be moulded from any suitable polymer. A cartridge or ampoule is preferably sealed at one end by a pierceable membrane referred to as the **"septum"** which can be pierced e.g. by the non-patient end of a needle cannula. Such septum is usually self-sealing which means that the opening created during penetration seals automatically by the inherent resiliency once the needle cannula is removed from the septum. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container - rigid or flexible - can be used to contain the drug.
Since a cartridge usually has a narrower distal neck portion into which the plunger cannot be moved not all of the liquid drug contained inside the cartridge can actually be expelled. The term **"initial quantum"** or **"substantially used"** therefore refers to the injectable content contained in the cartridge and thus not necessarily to the entire content.
By the term **"Pre-filled"** injection device is meant an injection device in which the cartridge containing the liquid drug is permanently embedded in the injection device such that it cannot be removed without permanent destruction of the injection device. Once the pre-filled amount of liquid drug in the cartridge is used, the user normally discards the entire injection device. This is in opposition to a **"Durable"** injection device in which the user can himself change the cartridge containing the liquid drug whenever it is empty. Pre-filled injection devices are usually sold in packages containing more than one injection device whereas durable injection devices are usually sold one at a time. When using pre-filled injection devices an average user might require as many as 50 to 100 injection devices per year whereas when using durable injection devices one single injection device could last for several years, however, the average user would require 50 to 100 new cartridges per year.
The term **"protective cap"** is herein meant to refer to a cover or a sleeve-like structure which is mounted at the distal end of the injection device between injections. Such cover or sleeve-like structure is closed at the distal end. The protective cap covers and protects the distal end of the injection device between injections. Should a needle cannula be mounted to the injection device, either permanently or exchangeable, such needle cannula is henceforth also protected by the protective cap. Since the protective cap is mounted each time an injection has been performed, the word "re-mounted" can also be used to describe the mounting of the protective cap. Further, since the housing structure of an injection device often has a window through which the user can inspect the liquid drug, the protective cap also protect the drug from long time exposure to light as some liquids drug are sensitive to light exposure, especially to UV light.

All headings and sub-headings are used herein for convenience only and should not be constructed as limiting the invention in any way.
The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.
This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1: show a cross sectional view of the protective cap according to a first example of the invention.
- Figure 2: show a perspective view of the axially movable and rotatable shield according to the first example.
- Figure 3: show a perspective view of the coupling between the housing structure and the protective cap according to the first example.
- Figure 4: show a perspective view of the axially movable shield in the first and locked position in relation to the housing structure.
- Figure 5: show a perspective view of the axially movable shield in the second and unlocked position in relation to the housing structure.
- Figure 6: show a perspective view of the engagement between the housing structure and the axially movable shield with the axially movable shield in the first rotatable position
- Figure 7: show a perspective view of the engagement between the housing structure and the axially movable shield with the axially movable shield in the second rotatable position.
- Figure 8: show a perspective view of the injection device according to a second example with the protective cap attached to the housing structure.
- Figure 9: show a perspective view of the injection device according to the second example with the protective cap dismounted.
- Figure 10: show a perspective view of the protective cap according to the second and third example.
- Figure 11: show a cross-sectional view of the distal part of the injection device with the axially movable shield in the first position.
- Figure 12: show a cross-sectional view of the distal part of the injection device with the axially movable shield in the second position.
- Figure 13: show a perspective view of the protective two-part cap.
- Figure 14: show a perspective view of the distal part and the proximal part making up the protective two-part cap.
- Figure 15: show a close-up view of the track configuration of the protective two part cap.
- Figure 16A: show a perspective view of the injection device of a third example wherein the protective cap (with parts visually cut away) is being axially mounted onto the housing structure.
- Figure 16B: show a side view of the injection device shown in figure 16A.
- Figure 17A: show a perspective view of the injection device according to the third example wherein the protective cap (with parts visually cut away) is being rotated relatively to the housing structure.
- Figure 17B: show a side view of the injection device shown in figure 17A.
- Figure 18A-B: show a side view of the protective two-part cap and the housing structure as the longitudinal driving rib inside the protective two-part cap drives the outwardly pointing protrusion of the axially movable shield during an initiation process.
- Figure 19A-B: show a side view of the protective two-part cap and the housing structure as the protective two-part cap is being axially removed from the housing structure.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as their relative dimensions are intended to serve illustrative purposes only.

In that context it may be convenient to define that the term "distal end" in the appended figures is meant to refer to the end of the injection device which usually carries the protective cap whereas the term "proximal end" is meant to refer to the opposite end pointing away from the protective cap and usually carrying the dose dial button.

As the figures are merely schematic representations some deviations between the different drawings, even within the same embodiments, can occur without influencing the claimed invention.

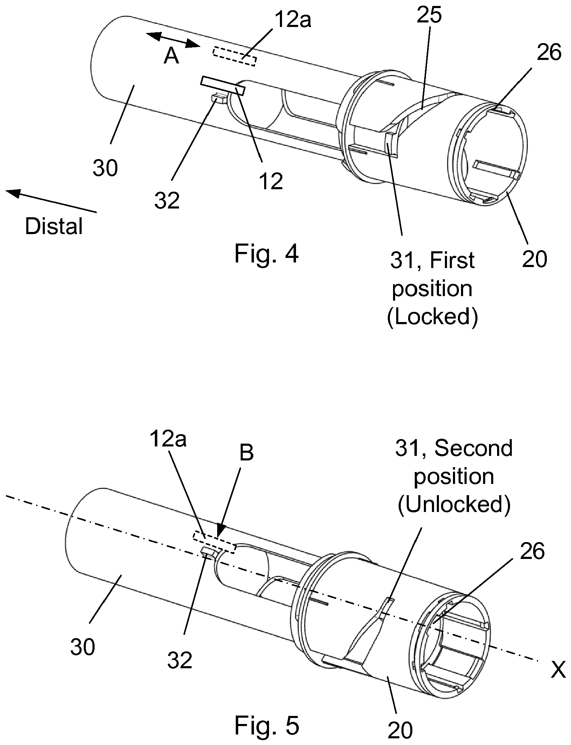

Distal and proximal are meant to be along an axial orientation extending along the longitudinal axis "X" of the injection device as further indicated in figure 4 and 5 which discloses the front end of the injection device according to a first embodiment.

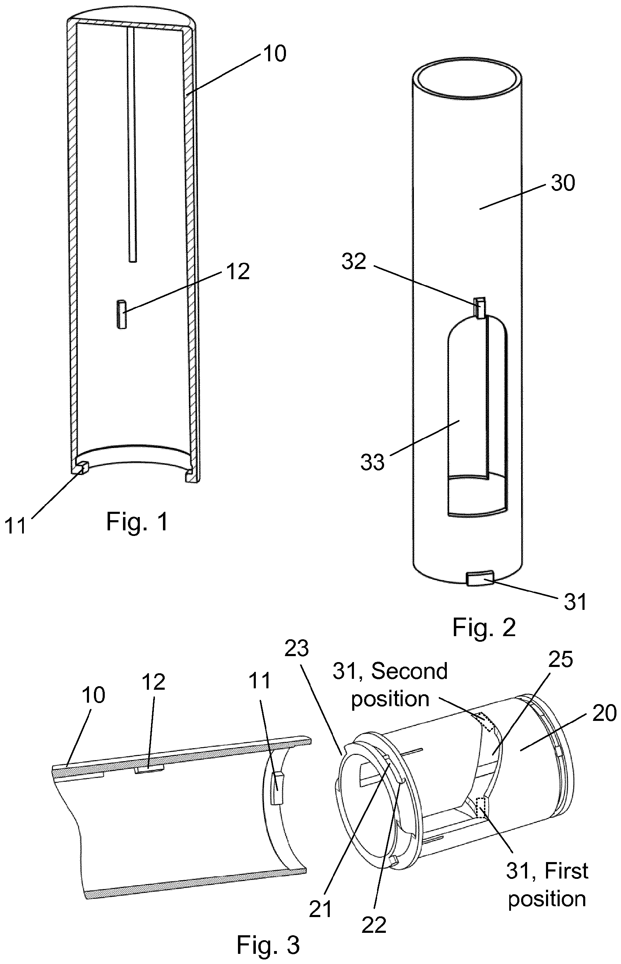

Figure 1 discloses the protective cap 10 which proximally is provided with a number of inwardly pointing protrusions 11 which engages the housing structure 20. Internally the protective cap 10 is provided with one or more longitudinal driving protrusions or ribs 12.

Whenever in the following reference is made to a rib, a track, a protrusion or similar element these can be provided in any number. Since the exemplified embodiments all concern a penshaped injection device with a circular cross section, the ribs, tracks and protrusions referred to are preferably supplied in pairs of two.

As best seen in figure 3, the housing structure 20 is provided with a number of peripheral tracks 21 in which the inwardly pointing protrusions 11 on the protective cap 10 are received. These peripheral tracks 21 are shaped such that the protective cap 10 most be rotated relatively to the housing structure 20 before the protective cap 10 can be removed from the engagement with the housing structure 20.

The housing structure 20 can either be formed as a single housing or a housing made from any plurality of individual parts coupled together to form a housing structure. Usually a housing structure 20 comprises a part securing the mechanical components of the dose setting and injection mechanism and another part securing the cartridge which contains the medicament to be injected. Such part is often referred to as the cartridge holder. However, other parts can also be involved in the design of the housing structure 20.

In the first embodiment disclosed in the figures 1 to 5, the housing structure 20 is visually shown as a separate part which is connected to the remaining parts of the housing as disclosed in European patent application Number 16-188751.8.

Figure 2 discloses the axially movable shield 30. The axially movable shield 30 which covers the distal tip of a needle cannula between injections has a guiding protrusion 31 located at the proximal periphery and an outwardly pointing protrusion 32 also provided on the outer surface. Further, a window 33 is provided for viewing the content of the injection device. Distally the axially movable shield 30 can be provided with a cleaning chamber for cleaning the distal tip of the needle cannula between injections. This cleaning chamber will be explained in connection with a later embodiment, but could easily be used for all embodiments..

When the injection device is delivered to the user, the protective cap 10 is pre-mounted by the manufacture onto the housing structure 20 and the inwardly pointing protrusion 11 is located in the peripheral track 21 abutting the end 22 of the peripheral track 21. Opposite to the end 22, the peripheral track 21 terminates in an axial opening 23 through which the inwardly pointing protrusion 11 can be axially removed as the protective cap 10 is removed.

In the first locked position disclosed in figure 4, the guiding protrusion 31 on the axially movable shield 30 is located in the first position as also indicated in the lower section of figure 3. In this first position, the axially movable shield 30 is prevented from moving in the proximal direction due to the abutment of the guiding protrusion 31 with the radial sidewall of the track 25.

The track 25 in the housing structure 20 which is engaged by the guiding protrusion 31 guides the axially movable shield 30 between the first position and the second position during rotation of the axially movable shield 30. As seen from the figures 4 and 5, the track 25 connects to an axial track 26 which in the disclosed embodiment is not cut through the housing structure 20 but provided on the inner surface of the housing structure 20.

In the figures 4 and 5 the position of the longitudinal driving rib 12 is indicated. When the user rotationally removes the protective cap 10 by anti-clockwise rotation as seen in figure 3, the inwardly pointing protrusion 11 is rotated to a position in which the protective cap 10 can be axially released from its engagement with the housing structure 20. During anti clock-wise rotation of the protective cap 10 in order to remove the protective cap 10, the longitudinal driving ribs 12 inside the protective cap 10 is moved to the position indicated with a broken line 12a in figure 4. In this position, the protective cap 10 can be axially removed as indicated by the arrow "A". During the rotation of the protective cap 10, the longitudinal driving rib 12 does not abut the axially movable shield 30 which thus remains in its initial rotational position (the locked position). The protective cap 10 is henceforth rotated to the axially releasable position while the axially movable shield 30 remains in its locked position.

Once the user has rotated the protective cap 10 and removed it axially along the arrrow "A", the user must manually rotate the axially movable shield 30 to the second (unlocked) position disclosed in the upper part of figure 3 and in figure 5. In this second unlocked position, the axially movable shield 30 is free to move axially as the guiding protrusion 31 moves axially in the axial track 26 and an injection can henceforth be performed.

Following the injection, the user must manually rotate the axial movable shield 30 back to the first position to lock the injection device. However, should the user forget to manually rotate the axial movable shield 30 this is done automatically when mounts the protective cap 10.

When the protective cap 10 is axially positioned on the housing structure 20, the longitudinal driving rib 12 is in the position 12a indicated in figure 4 and 5. If the axial movable shield 30 has not been rotated back to the locked position, the outwardly pointing protrusion 32 is in the position disclosed in figure 5. From this position, the user rotates the protective cap 10 clock-wise such that the inwardly pointing protrusion 11 moves peripherally in the peripheral track 21. This rotation of the protective cap 10 and henceforth the longitudinal driving rib 12 moves the outwardly pointing protrusion 32 in the clockwise direction as indicated with the arrow "B" in figure 5. As the axial movable shield 30 rotate back to the first and locked position, the guiding protrusion 31 moves clockwise in the track 25.

### Second Embodiment:

In the second embodiment disclosed in figure 6 to 12, similar constructive elements are provided with the same reference numeral, however, with a "1" in front. The housing structure in this second embodiment is thus indicated as "120" and the axially movable shield is numbered "130". As previously mentioned such housing structure can encompass a cartridge holder which in this second embodiment is referred to as "120A". Henceforth, in this second embodiment the housing structure 120 comprises the cartridge holder 120A and a proximal housing part 120B.

The cartridge holder 120A is provided with one or more protrusions 125 which secure and guide the axially movable shield 130 as will be explained. The protrusion 125 is guided in a track 135 which comprises an axial part 136, a helical part 137 and a radial part 138.

When the injection device is delivered to the user, the protrusion 125 is positioned in the radial part 138 of the track 135 as disclosed in figure 6. In this initial position, the axially movable shield 130 is prevented from axial movement as the protrusion 125 cannot move axially in the radial track 138.

In order to neutralize the pressure in the liquid system, the user needs to rotate the axially movable shield 130 a predetermined angle defined by the track 135 as indicated by the arrow "A" in figure 6. This rotation moves the protrusion 125 through the helical part 137 of the track 135 and into the axial part 136 of the track 135.

All movements of the protrusion 125 herein described are relative movements between the protrusion 125 and the track 135. As shown in the second embodiment, the protrusion 125 is provided on the cartridge holder 120A and the track 135 is provided in the axially movable shield 130, however opposite placement as disclosed in the first embodiment generates the same effect.

As the protrusion 125 moves through the helical part 137 of the track 135, the axially movable shield 130 is moved backward in the proximal direction a distance as indicated by the arrow "B" in figure 7. This distance is determined by the helical pitch of the helical part 137 of the track 135.

The axially movable shield 130 is thus moved rotationally and axially (i.e. helically) relatively to the cartridge holder 120A and hence the housing structure 120 by an angle defined by the track 135. Further, an additional protrusion 128 engaging a window 133 for limiting the angle of rotation can be provided on the housing structure 120 and preferably on the cartridge holder 120A of the housing structure 120.

The window 133 in the axially movable shield 130 can further be aligned with a similar longitudinal opening 127 provided in the cartridge holder 120A such that the liquid drug inside the cartridge 105 can only be viewed when the window 133 is aligned with the longitudinal opening 127 as disclosed in figure 7. This further indicates to the user that the injection device is in a position ready to perform an injection.

The outer surface of the axially movable shield 130 is further provided with an outwardly pointing protrusion 132 which is engaged by the protective cap 110 in order to rotate the axially movable shield 130 back to the first locked position should the user forget to do this manually. This outwardly pointing protrusion 132 can be formed as a regular protrusion as depicted in figure 7 or alternatively as a longitudinal rib as depicted in figure 9. The engaging surface on the protective cap 110 is also in this embodiment formed as an inwardly pointing longitudinal rib structure 112 as indicated in figure 10.

A more detailed view of the injection device as partly disclosed in figure 6 and 7 are provided in the figures 8 to 12 wherein figure 8 and figure 9 are perspective views of the injection device according to the second embodiment. However, minor deviations in the individual drawings can occur.

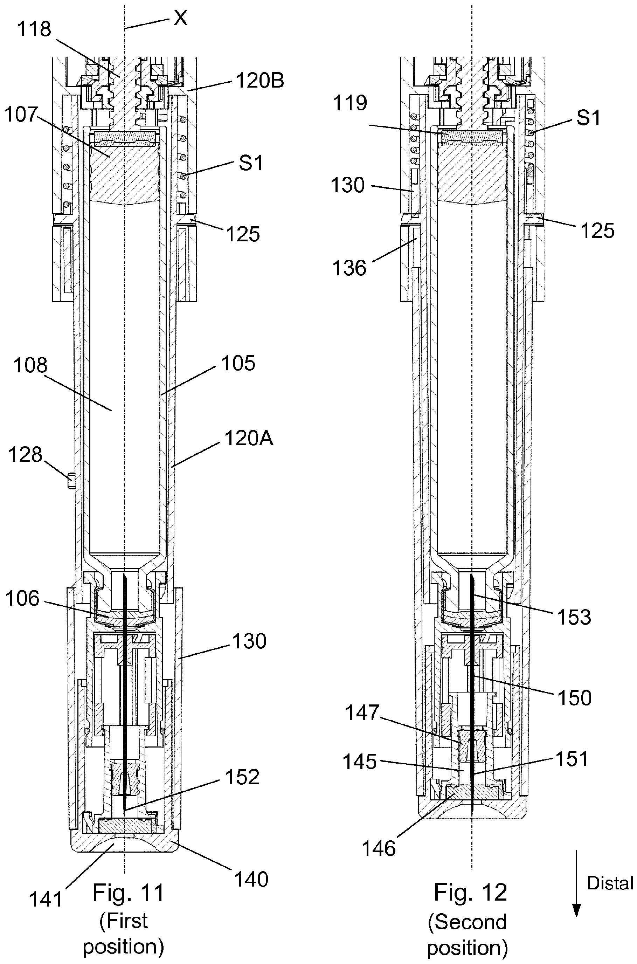

The housing structure 120 is proximally provided with a dose setting button 115 and distally with the axially movable shield 130. The protective cap 110, which has been removed in the figures 11 and 12 covers the distal end of the injection device during storage and is usually only removed when the user performs an injection.

As best seen in figure 9, the proximal housing part 120B of the housing structure 120 is externally provided with a protrusion 121 which engages a helical track 111 located inside the protective cap 110 as best seen in figure 10. Since the track 111 is helical, the user needs to rotate the protective cap 110 relatively to the housing structure 120 in order to remove the protective cap 110.

The protective cap 110 which in the first embodiment was moulded as one unitary unit is in this second embodiment formed from two parts 110A, 110B which in one example are moulded in a 2K moulding. Alternatively, the protective cap 10 can be made from two individual and separately moulded parts which are click-fitted together as will be explained in the third embodiment.

In figure 9, the protective cap 110 has been removed by rotating the protective cap 110 relatively to the protrusion 121 on the proximal housing part 120B. Further, in figure 9, the axially movable shield 130 has been rotated to the second unlocked position and the distal part 151 of the needle cannula 150 is thus visible.

As further disclosed in figure 9, the axially movable shield 130 is distally provided with a cleaning unit 140 which are shown in more details on figure 11 and 12. Further, on figure 9, the outwardly pointing protrusion 132 provided on the axially movable shield 130 is depicted as a longitudinal rib.

The figures 11 and 12 disclose a cross sectional view of the distal end of injection device of the second embodiment with the protective cap 110 fully removed.

The cartridge holder 120A is secured to the proximal housing part 120B by having the protrusions 125 penetrate further though holes in the proximal housing part 120B. The cartridge-holder 120A secures the cartridge 105 which contain a liquid drug. This liquid drug preferably contains a preservative such as meta-cresol, phenol or similar preservative.

The cartridge 105 is typically a hollow cylinder-shaped container which is distally provided with a pierceable septum 106 and proximally provided with a movable plunger 107 which can be moved in the distal direction to force the liquid drug out of the cartridge 105 through the needle cannula 150 penetrated through the distal septum 106. The pierceable septum 106 and the movable plunger 107 thus define an interior 108 containing the liquid drug.

The needle cannula 150 has a distal end 151 with a distal tip 152 and a proximal end 153 which are connected via a lumen through which the liquid drug flows. The distal tip 152 penetrates through the skin of the user during injection and the proximal end 153 penetrates into the cartridge 105.

The proximal housing part 120B holds the dose setting and injecting mechanism which could be any kind of manually working or spring operated mechanism. The dose setting and injecting mechanism moves a piston rod 118 in the distal direction during injection and the piston rod 118 abuts and moves the plunger 107 forward inside the cartridge 105. Usually a piston rod foot 119 is provided between the piston rod 118 and the plunger 107 to distribute the force

On the outside surface of the cartridge holder 120A, a axially movable shield 130 is provided. This axially movable shield 130 is movable relatively to the cartridge holder 120A between a first position and a second position and since the cartridge holder 120A is permanently connected to the proximal housing part 120B, the axially movable shield 130 also moves relatively to the proximal housing part 120B and thus to the entire housing structure 120. A compression spring S1 is located between the proximal housing part 120B and the axially movable shield 130. The purpose of this compression spring S1 is to return the axially movable shield 130 to its initial position following an injection.

Distally the axially movable shield 130 carries a cleaning unit 140 which comprises a cleaning chamber 145 containing a cleaning solvent for cleaning the distal tip 152 of the needle cannula 150 between subsequent injections. The cleaning solvent contained in the cleaning chamber 145 is preferably an amount of the liquid drug contained in the cartridge 105 as explained in WO2015/062845. Typically, such liquid drug contains a preservative such as meta-cresol or phenol or a combination thereof which thus acts as the cleaning solvent. The liquid drug can be any kind of injectable liquid drug but is preferably a blood sugar regulating liquid drug such as insulin, insulin analogue, GLP-1 or GLP-2 or a combination of any of these liquid drugs.

Through an initialization process as described in WO 2016/173895, a predetermined amount of the liquid drug contained in the cartridge 105 is transferred to the cleaning chamber 145 which is defined between the seal 146 and the piston 147.

The initialization process has two steps. First the needle cannula 150 is moved into the position depicted in figure 11. In this position, the proximal end 153 of the needle cannula 150 is penetrated into the interior 108 of the cartridge 105 and the distal tip 152 is positioned inside the cleaning chamber 145. Further, in the following step of the initialization process an amount of the liquid drug is pumped from the cartridge 105 and into the cleaning chamber 145 by pressurizing the liquid drug inside the cartridge 105 either by moving the plunger 107 forward or by moving the cartridge 105 proximally while maintaining the position of the plunger 107.

However, when pressurizing the interior 108 of the cartridge 105, the pressure applied is often larger than the pressure actually needed to make sure the cleaning chamber 145 is adequately filled. As a result an overpressure will be present in the liquid system comprising the cartridge 105, the lumen of the needle cannula 150 and the cleaning chamber 145.

Before performing an injection it is thus necessary to neutralize this overpressure as explained in European patent application No. EP 15 182290.5. For this purpose, the cartridge holder 120A is provided with the protrusion 125 engaging the track 135.

In the first position of the axially movable shield 130, as disclosed in figure 11 (and figure 6), the protrusion 125 is positioned in the radial part 138 of the track 135. The axially movable shield 130 is thus prevented from axial movement.

When the user rotates the axially movable shield 130 relatively to the housing structure 120, the protrusion 125 is moved through the helical part 137 of the track 135 and into the axial part 136.

In this second position which is depicted in figure 7, figure 9 and figure 12, the axially movable shield 130 has been retracted such that the distal tip 152 of the needle cannula 150 is positioned in front of the axially movable shield 130 but preferably within a concave recess 141 provided distally in the cleaning unit 140 secured to the axially movable shield 130. The cleaning unit 140 can also be an integral part of the axially movable shield 130. When the protrusion 125 is positioned in the axial part 136 of the track 135 which is best seen in figure 7, the axially movable shield 130 can be moved proximally in relation to the housing structure 120 and an injection can be performed.

Once an injection has been performed, the user removes the axially movable shield 130 from the skin of the user, and the spring S1 automatically returns the axially movable shield 130 to the second position disclosed in figure 12. From this position, the user can once again lock the axial movement of the axially movable shield 130 by rotating the axially movable shield 130. Such rotation moves the axially movable shield distally due to the helical part 137 of the track 135 where after the distal tip 152 of the needle cannula 150 is once again positioned inside the cleaning chamber 145 as disclosed in figure 11.

### Third Embodiment:

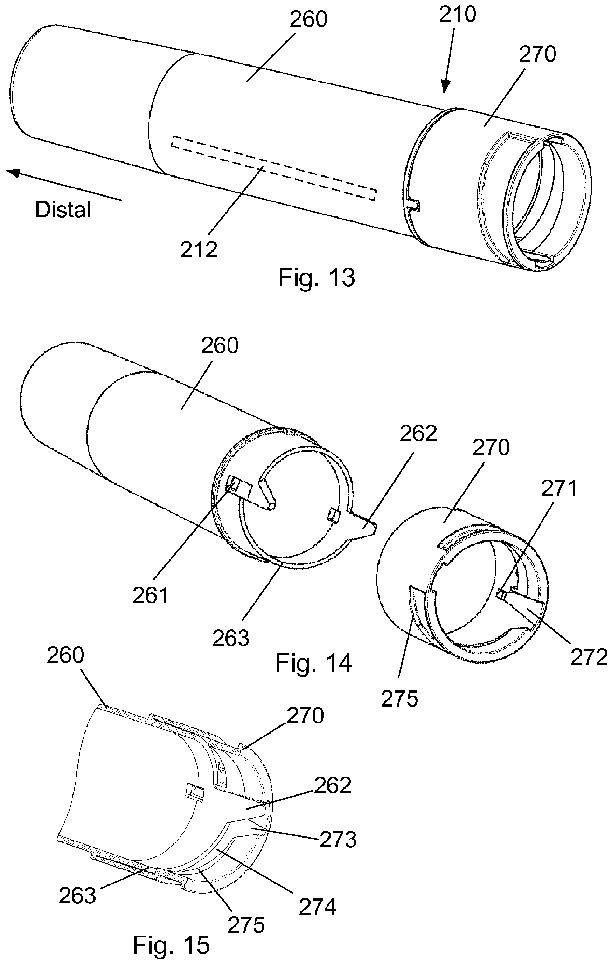

The third embodiment explains the two-part protective cap as disclosed in the figures 13 - 19B. Similar constructive elements are provided with the same reference numeral, however, with a "2" in front. The protective cap is henceforth in this third embodiment referred to as "210".

As seen in the figures 13 to 15, the protective cap 110 is formed from two different and separate parts; a distal part 260 and a proximal part 270. The distal part 260 is the actual cap and the proximal part 270 is a ring shaped element as best seen in figure 14.

The distal part 260 has a number of openings 261 into which a similar number of radial protrusions 271 provided on the inner surface of the proximal part 270 fits such that the two parts 260, 270 can be clicked together to form one operational protective cap 210.

The distal part 260 is further provided with a number of guiding noses 262 which slides into axial tracks 272 in the proximal part 270. When the distal part 260 and the proximal part 270 are click fitted together, a part of the axial track 272 is left open to form an axial guiding track or opening 273 as best seen in figure 15.

The protective cap 210 is also in the second embodiment provided with a driving rib 212 provided on the inner surface as indicated by punctured lines in figure 13.

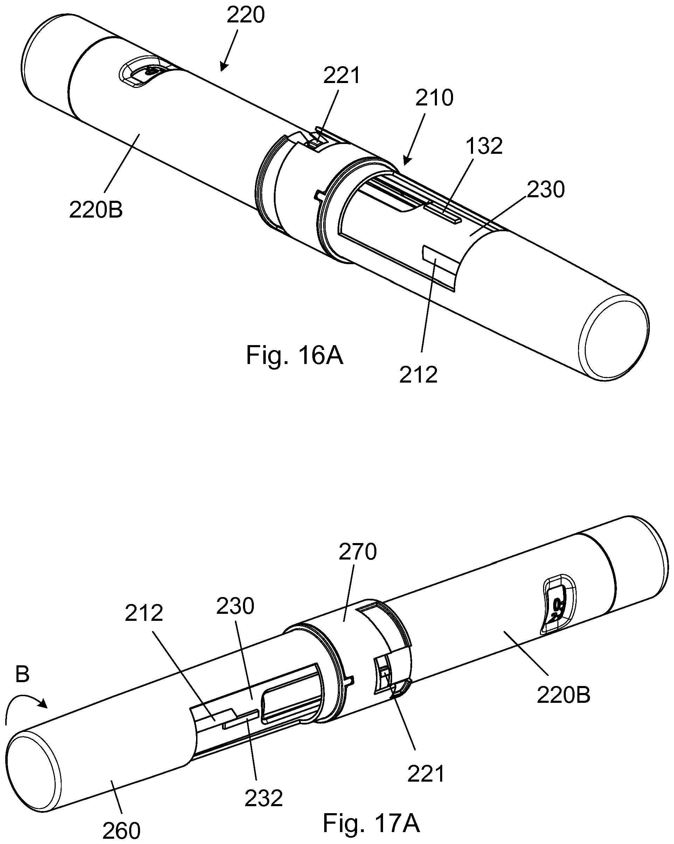

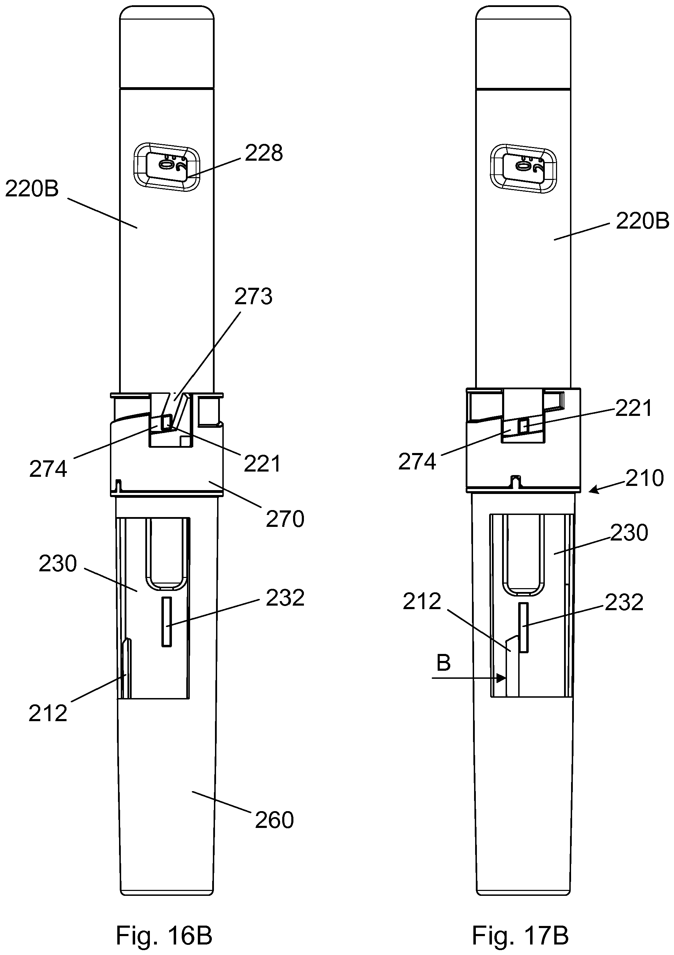

This axial guiding track 273 fits over outwardly pointing protrusions 221 provided on the proximal housing part 220B of the housing structure 220 as best seen in figure 16A and 16B. Also in this embodiment does the housing structure 220 comprise of a not-shown cartridge holder and a proximal housing part 220B. The guiding track 273 further leads to a peripheral track 274 which are provided between the distal part 260 and the proximal part 270 as best seen in figure 15.

As seen in figure 14 and figure 15, the peripheral track 274 is provided between the proximal end surface 263 of the distal part 260 and a mould surface 275 provided in the proximal part 270.

When the protective cap 210 is being mounted or removed from the housing structure 220, the outwardly pointing housing protrusion 221 moves in this peripheral track 274 which forces the user to rotate the protective cap 210 relatively to the housing structure 220 before the outwardly pointing housing protrusion 221 can enter the axial track 273 and the protective cap 210 can be mounted or removed. The peripheral track 274 can as depicted in figure 15 be slightly helical.

In figure 16A-B and 17A-B parts of the protective cap 210 have been visually cut away in order to view the different protrusions and ribs.

As in the first embodiment, the user, after having removed the protective cap 210, rotates the axially movable shield 230 to an unlocked position and performs an injection. Should the user forget to manually rotate the axially movable shield 230 back to the locked position this is done automatically upon mounting of the protective cap 210 as will be explained in conjunction with figure 16A-B and figure 17A-B.

Figure 16A-B discloses the state in which the axially movable shield 230 is in the second and unlocked position. The user mounts the protective cap 210 by inserting the track 273 over the outwardly pointing housing protrusion 221. In this position, the longitudinal driving rib 212 is placed in an anti-clockwise position in relation to the outwardly pointing protrusion 232 on the axially movable shield 230.

When the user starts to rotate the protective cap 210 in the clockwise direction as indicated by the arrow "B" in the figure 17A-B, the longitudinal driving rib 212 abuts the outwardly pointing protrusion 232 on the axially movable shield 230.

Figure 17B depicts the situation in which the outwardly pointing housing protrusion 221 is positioned approximately halfway in the peripheral track 274 and further rotation forces the outwardly pointing protrusion 232 and thus the axially movable shield 230 clockwise back into the first and locked position.

In the first embodiment, the peripheral track 21 was formed as a purely radial track whereas in the second and third embodiments the peripheral track 274 is formed as a helical track such that the protective cap 210 is also moved axially during mounting and removal of the protective cap 210.

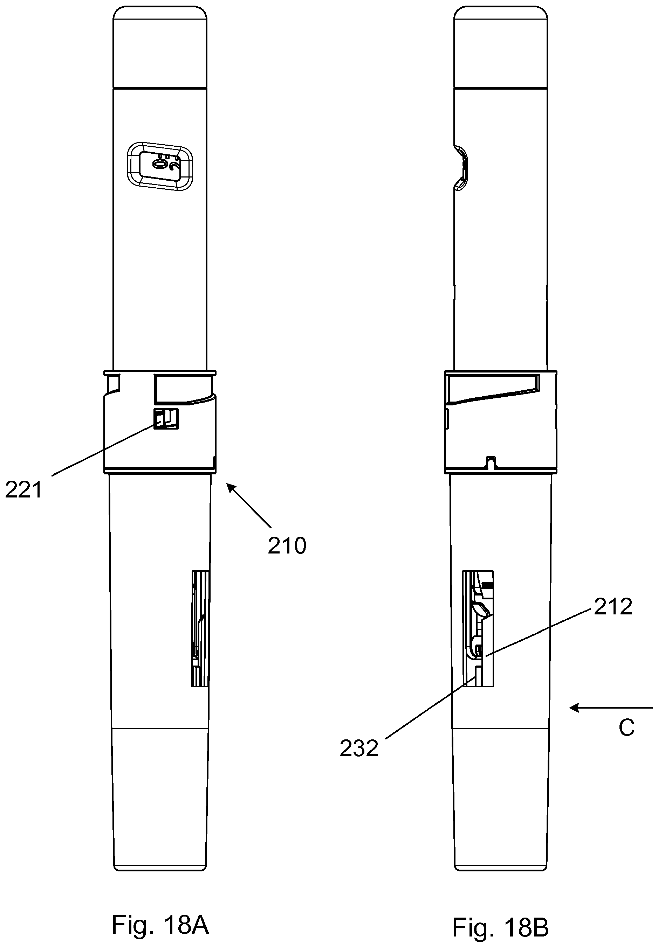

However, in some situations it is preferred to have the longitudinal driving rib 212 located in a clockwise position relatively to the outwardly pointing protrusion 132. This is e.g. disclosed in figure 18A-B.

In figure 18A the outwardly pointing housing protrusion 221 is located at the end of the peripheral track 274 furthest away from the axial guiding track 273. When the user rotates the protective cap 210 in the in the anti-clockwise direction indicated by the arrow "C" in figure 18B to remove the protective cap210, the longitudinal driving rib 212 abuts the outwardly pointing protrusion 232 and rotates the axially movable shield 230. This initial rotation of the axially movable shield 230 can e.g. be used in an initiation process of the injection device as explained in European patent application number EP 16-188751.8.

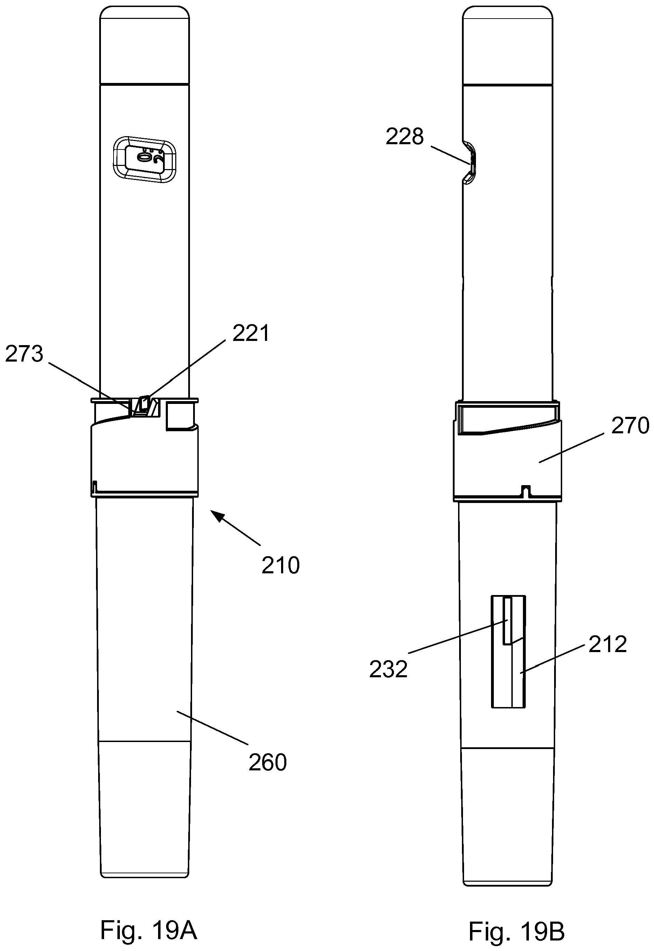

Figure 19A-B discloses the position in which the protective cap 210 has been rotated to a position in which the outwardly pointing housing protrusion 221 can slide axially through the axial guiding track 273. The user is thus able to axially remove the protective cap 210.

Once the protective cap 210 is removed, the user rotates the axially movable shield 230 to unlock the axially movable shield 230. The axially movable shield 230 is thus rotated from the position depicted in figure 19B to the position disclosed in figure 16B.

In the view in the figures 18B and 19B the injection device as a whole has been rotated 90 degrees in the clockwise direction. This can e.g. be seen by the position of the viewing window 228.

When the user mounts the protective cap 210, the longitudinal driving rib 221 will thus be on the anti-clockwise side of the outwardly pointing protrusion 232 as depicted in figure 16B.

If the longitudinal driving rib 221 is to be initial located in the clockwise position relatively to the outwardly pointing protrusion 232 as depicted in figure 11A-B and figure 12A-B, it is necessary to split the protective cap 210 in two parts such as a distal part 260 and a proximal part 270.

When initially mounting the two-part protective cap 210 onto the housing structure 220 during manufacturing of the injection device, the proximal part 270 is first placed in the position depicted in figure 18A with the outwardly pointing housing protrusion 221 located at the end of the peripheral track 274.

Following this, the distal part 260 is slid axially into its click-fit connection with the proximal part 270 such that the longitudinal driving rib 212 is positioned clockwise to the outwardly pointing protrusion 32 as disclosed in figure 18B.

When the user initially rotates the protective cap 210 in order to remove it, this anti-clockwise rotation will be transformed into a rotation of the axially movable shield 232.

Only when assembling the two parts 260, 270 of the protective cap 210 in this order will it be possible to have the longitudinal driving rib 212 to be positioned such in relation to the outwardly pointing protrusion 232 that an initial rotation of the axially movable shield 230 will be performed upon first rotation and removable of the protective cap 210.

Some preferred embodiments have been shown in the foregoing, but it should be stressed that the invention is not limited to these, but may be embodied in other ways within the subject matter defined in the following claims.

## Claims

1. An injection device for apportioning set doses of a liquid drug comprising
a housing structure (20, 120, 220) supporting a non-removable cartridge (105) having an interior chamber (108) containing the liquid drug to be injected,
A needle cannula (150) supported by a distal end of the housing structure (20, 120, 220) and having a proximal end (153) inserted into the interior chamber (108) of the cartridge (105) and a distal end (151) with a distal tip (152),
an axially movable shield (30, 130, 230) for covering the distal tip (152) of the needle cannula (150) between injections, which axially movable shield (30, 130, 230) is rotatable relatively to the housing structure (20, 120, 230) between a first locked position and a second unlocked position,
- the first locked position being a position in which the axially movable shield (30, 130, 230) is prevented from moving proximally, and
- the second unlocked position being a position in which the axially movable shield (30, 130, 230) is allowed to move proximally,
***characterized in that*** the axially movable shield (30, 130, 230) distally is provided with a cleaning chamber (145) for cleaning at least the distal tip (152) of the needle cannula (150) between injections,
a removable protective cap (10, 110, 210) closed at a distal end, and coupled to the housing structure (20, 120, 220) for covering at least a distal part of the housing structure (20, 120, 220) and the distal tip (152) of the needle cannula (150), and which removable protective cap (10, 110, 210) is rotatably coupled to the housing structure (20, 120, 220) requiring a user to rotate the protective cap (10, 110, 210) relatively to the housing structure (20, 120, 220) to allow removal of the protective cap (10, 110, 210) and which protective cap (10, 110, 210) comprises one or more protrusions or longitudinal structures such as ribs (12, 112, 212) on an inner surface Z of the removable protective cap (10, 110, 210) for rotationally engaging a number of outwardly pointing protrusions or the like (32, 132, 232) provided on the axially movable shield (30, 130, 230) such that the required rotation of the removable protective cap (10, 110, 210) relative to the housing structure (20, 120, 220) is transformed to a rotation of the axially movable shield (30, 130, 230).

2. An injection device according to claim 1, wherein the protective cap (10) is provided with one or more inwardly pointing protrusions (11) and the housing structure (20) is provided with one or more peripheral tracks (21) in which the inwardly pointing protrusions (11) are guided.

3. An injection device according to claim 2, wherein the peripheral tracks (21) provided in the housing structure (20) terminate in radial openings (23).

4. An injection device according to claim 1, wherein the protective cap (110, 210) is provided with one or more peripheral tracks (111, 274) in which one or more outwardly pointing protrusions (121, 221) provided on the housing structure (120) are guided.

5. An injection device according to claim 4, wherein the peripheral tracks (111, 274) provided in the protective cap (110, 210) terminate in radial openings (273).

6. An injection device according to claim 4 or 5, wherein the protective cap (110, 210) comprises a distal part (110B, 260) and a proximal part (110A, 270).

7. An injection device according to claim 6, wherein the distal part (110B, 260) and the proximal part (110A, 270) are click fitted together to form one operational protective cap (110, 210).

8. An injection device according to claim 6 or 7, wherein the peripheral track (111, 274) is provided in the junction between the distal part (110B, 260) and the proximal part (110A, 270).

9. An injection device according to claim 6, 7 or 8, wherein the proximal part (110A, 270) is mounted onto the housing structure (120, 220) and the distal part (110B, 260) is axially connected to the proximal part (150).

10. An injection device according to any of the previous claims, wherein the distal tip (152) of the needle cannula (150) is located inside the cleaning chamber (145) when the axially movable shield (30, 130, 230) is in the first locked position, and the distal tip (152) is located outside and distal to the cleaning chamber (145) when the axially movable shield (30, 130, 230) is in the second unlocked position.

## Patentansprüche

1. Injektionsvorrichtung zum Aufteilen eingestellter Dosen eines flüssigen Arzneimittels, umfassend:
eine Gehäusestruktur (20, 120, 220), die eine nicht entfernbare Kartusche (105) trägt, die eine Innenkammer (108) aufweist, die das zu injizierende flüssige Arzneimittel enthält,
eine Nadelkanüle (150), die von einem distalen Ende der Gehäusestruktur (20, 120, 220) getragen wird und ein proximales Ende (153), das in die Innenkammer (108) der Kartusche (105) eingeführt ist, und ein distales Ende (151) mit einer distalen Spitze (152) aufweist,
eine axial bewegliche Blende (30, 130, 230) zum Bedecken der distalen Spitze (152) der Nadelkanüle (150) zwischen Injektionen, wobei die axial bewegliche Blende (30, 130, 230) im Verhältnis zu der Gehäusestruktur (20, 120, 230) zwischen einer ersten verriegelten Position und einer zweiten entriegelten Position drehbar ist,
- wobei die erste verriegelte Position eine Position ist, in der die axial bewegliche Blende (30, 130, 230) daran gehindert wird, sich proximal zu bewegen,
- wobei die zweite entriegelte Position eine Position ist, in der die axial bewegliche Blende (30, 130, 230) sich proximal bewegen kann, und
**dadurch gekennzeichnet, dass** die axial bewegliche Blende (30, 130, 230) distal mit einer Reinigungskammer (145) zum Reinigen zumindest der distalen Spitze (152) der Nadelkanüle (150) zwischen Injektionen, bereitgestellt ist,
eine entfernbare Schutzkappe (10, 110, 210), die an einem distalen Ende geschlossen und mit der Gehäusestruktur (20, 120, 220) gekoppelt ist, um zumindest einen distalen Teil der Gehäusestruktur (20, 120, 220) und die distale Spitze (152) der Nadelkanüle (150) zu bedecken, wobei die entfernbare Schutzkappe (10, 110, 210) drehbar mit der Gehäusestruktur (20, 120, 220) verbunden ist, was erfordert, dass ein Benutzer die Schutzkappe (10, 110, 210) im Verhältnis zu der Gehäusestruktur (20, 120, 220) dreht, um das Entfernen der Schutzkappe (10, 110, 210) zu ermöglichen, und wobei die Schutzkappe (10, 110, 210) einen oder mehrere Vorsprünge oder in Längsrichtung verlaufende Strukturen, wie etwa Rippen (12, 112, 212), auf einer Innenfläche der entfernbaren Schutzkappe (10, 110, 210) zum drehbaren Eingreifen in eine Anzahl von nach außen gerichteten Vorsprüngen oder dergleichen (32, 132, 232), die an der axial beweglichen Blende (30, 130, 230) bereitgestellt sind, umfasst, sodass die erforderliche Drehung der entfernbaren Schutzkappe (10, 110, 210) im Verhältnis zu der Gehäusestruktur (20, 120, 220) zu einer Drehung der axial beweglichen Blende (30, 130, 230) transformiert wird.

2. Injektionsvorrichtung nach Anspruch 1, wobei die Schutzkappe (10) mit einem oder mehreren nach innen gerichteten Vorsprüngen (11) bereitgestellt ist und die Gehäusestruktur (20) mit einer oder mehreren peripheren Schienen (21) bereitgestellt ist, die die nach innen gerichteten Vorsprünge (11) führen.

3. Injektionsvorrichtung nach Anspruch 2, wobei die peripheren Schienen (21), die in der Gehäusestruktur (20) bereitgestellt sind, in radialen Öffnungen (23) enden.

4. Injektionsvorrichtung nach Anspruch 1, wobei die Schutzkappe (110, 210) mit einer oder mehreren peripheren Schienen (111, 274) bereitgestellt ist, in denen eine oder mehrere an der Gehäusestruktur (120) bereitgestellte, nach außen gerichtete Vorsprünge (121, 221) geführt werden.

5. Injektionsvorrichtung nach Anspruch 4, wobei die in der Schutzkappe (110, 210) bereitgestellten peripheren Schienen (111, 274) in radialen Öffnungen (273) enden.

6. Injektionsvorrichtung nach Anspruch 4 oder 5, wobei die Schutzkappe (110, 210) einen distalen Teil (110B, 260) und einen proximalen Teil (110A, 270) umfasst.

7. Injektionsvorrichtung nach Anspruch 6, wobei der distale Teil (110B, 260) und der proximale Teil (110A, 270) ineinander eingerastet werden, um eine betriebsbereite Schutzkappe (110, 210) zu bilden.

8. Injektionsvorrichtung nach Anspruch 6 oder 7, wobei die periphere Schiene (111, 274) an der Verbindung zwischen dem distalen Teil (110B, 260) und dem proximalen Teil (110A, 270) bereitgestellt ist.

9. Injektionsvorrichtung nach Anspruch 6, 7 oder 8, wobei der proximale Teil (110A, 270) an der Gehäusestruktur (120, 220) befestigt ist und der distale Teil (110B, 260) axial mit dem proximalen Teil (150) verbunden ist.

10. Injektionsvorrichtung nach einem der vorhergehenden Ansprüche, wobei die distale Spitze (152) der Nadelkanüle (150) sich innerhalb der Reinigungskammer (145) befindet, wenn sich die axial bewegliche Blende (30, 130, 230) in der ersten verriegelten Position befindet, und sich die distale Spitze (152) außerhalb und distal zu der Reinigungskammer (145) befindet, wenn sich die axial bewegliche Blende (30, 130, 230) in der zweiten entriegelten Position befindet.

## Revendications

1. Dispositif d'injection pour la répartition de doses définies d'un médicament liquide comprenant
une structure de boîtier (20, 120, 220) supportant une cartouche non amovible (105) ayant une chambre intérieure (108) contenant le médicament liquide à injecter,
une canule d'aiguille (150) supportée par une extrémité distale de la structure de boîtier (20, 120, 220) et ayant une extrémité proximale (153) insérée dans la chambre intérieure (108) de la cartouche (105) et une extrémité distale (151) avec une pointe distale (152),
une protection axialement mobile (30, 130, 230) pour couvrir la pointe distale (152) de la canule d'aiguille (150) entre des injections, cette protection axialement mobile (30, 130, 230) étant rotative relativement à la structure de boîtier (20, 120, 230) entre une première position verrouillée et une deuxième position déverrouillée,
- la première position verrouillée étant une position dans laquelle la protection axialement mobile (30, 130, 230) est empêchée de se déplacer de manière proximale,
- la deuxième position déverrouillée étant une position dans laquelle la protection mobile (30, 130, 230) peut se déplacer de manière proximale, et
**caractérisé en ce que** la protection axialement mobile (30, 130, 230) est pourvue d'une chambre de nettoyage (145) pour nettoyer au moins la pointe distale (152) de la canule d'aiguille (150) entre des injections,
un capuchon de protection amovible (10, 110, 210) fermé à l'extrémité distale, et couplé à la structure de boîtier (20, 120, 220) pour couvrir au moins une partie distale de la structure de boîtier (20, 120, 220) et la pointe distale (152) de la canule d'aiguille (150), et lequel capuchon de protection amovible (10, 110, 210) est couplé en rotation à la structure de boîtier (20, 120, 220) exigeant d'un utilisateur de faire tourner le capuchon de protection (10, 110, 210) relativement à la structure de boîtier (20, 120, 220) pour permettre un retrait du capuchon de protection (10, 110, 210) et lequel capuchon de protection (10, 110, 210) comprend au moins une saillie ou structure longitudinale telle qu'une nervure (12, 112, 212) sur une surface interne du capuchon de protection amovible (10, 110, 210) pour un engagement en rotation d'un nombre de saillie pointant vers l'extérieur ou similaires (32, 132, 232) prévues sur la protection axialement mobile (30, 130, 230) de sorte que la rotation exigée du capuchon de protection amovible (10, 110, 210) par rapport à la structure de boîtier (20, 120, 220) soit transformée en une rotation de la protection axialement mobile (30, 130, 230).

2. Dispositif d'injection selon la revendication 1, dans lequel le capuchon de protection (10) est muni d'au moins une saillie pointant vers l'intérieur (11) et la structure de boîtier (20) est munie d'au moins une piste périphérique (21) dans laquelle les saillies (11) pointant vers l'intérieur sont guidée.

3. Dispositif d'injection selon la revendication 2, dans lequel les pistes périphériques (21) prévues dans la structure de boîtier (20) se terminent en ouvertures radiales (23).

4. Dispositif d'injection selon la revendication 1, dans lequel le capuchon de protection (110, 210) est muni d'au moins une piste périphérique (111, 274) dans laquelle au moins une saillie pointant vers l'extérieur (121, 221) prévue sur la structure de boîtier (120) est guidée.

5. Dispositif d'injection selon la revendication 4, dans lequel les pistes périphériques (111, 274) prévues dans le capuchon de protection (110, 210) se terminent en ouvertures radiales (273).

6. Dispositif d'injection selon la revendication 4 ou 5, dans lequel le capuchon de protection (110, 210) comprend une partie distale (110B, 260) et une partie proximale (110A, 270).

7. Dispositif d'injection selon la revendication 6, dans lequel la partie distale (110B, 260) et la partie proximale (110A, 270) sont adaptées par encliquetage ensemble pour former un capuchon de protection opérationnel (110, 210).

8. Dispositif d'injection selon la revendication 6 ou 7, dans lequel la piste périphérique (111, 274) est prévue dans la jonction entre la partie distale (110B, 260) et la partie proximale (110A, 270).

9. Dispositif d'injection selon la revendication 6, 7 ou 8, dans lequel la partie proximale (110A, 270) est montée sur la structure de boîtier (120, 220) et la partie distale (110B, 260) est raccordée axialement à la partie proximale (150).

10. Dispositif d'injection selon l'une quelconque des revendications précédentes, dans lequel la pointe distale (152) de la canule d'aiguille (150) est située à l'intérieur de la chambre de nettoyage (145) lorsque la protection axialement mobile (30, 130, 230) est dans la première position verrouillée, et la pointe distale (152) est située à l'extérieur et à l'extérieur de la chambre de nettoyage (145) et distalement à celle-ci lorsque la protection axialement mobile (30, 130, 230) est dans la deuxième position déverrouillée.
